# EUROPÄISCHE PATENTANMELDUNG

(11) **EP 3 284 406 A1**
(43) Veröffentlichungstag der Anmeldung: **21.02.2018**
(21) Anmeldenummer: 16184553.2
(22) Anmeldetag: 17.08.2016
(51) Int. Cl.: A61B 5/107, G06F 19/00, G01B 3/10

(54) **PÄDIATRISCHER NOTFALLRECHNER**

(71) Anmelder: Universität Zürich, 8006 Zürich (CH)
(72) Erfinder: WEISS, Markus, 7031 Laax (CH); SCHMIDT, Alexander, 8051 Zürich (CH)
(74) Vertreter: Schulz Junghans Patentanwälte PartGmbB

(57) **Zusammenfassung**

Die Erfindung betrifft eine Einrichtung zur Berechnung des Gewichtes und des Alters eines Kindes, insbesondere in einer medizinischen Notfallsituation, wobei die Einrichtung aufweist: ein Gehäuse; eine Längenmesseinheit, die zum Messen der Länge des Körpers des Kindes ausgebildet ist; eine im Gehäuse angeordnete elektronische Recheneinheit, die dazu konfiguriert ist unter Verwendung der gemessenen Länge eine automatische Berechnung des Gewichtes und/oder des Alters des Kindes unter Berücksichtigung des Geschlechtes, des Habitus und der Ethnizität vorzunehmen.

## Beschreibung

Die vorliegende Erfindung betrifft eine Einrichtung zur Berechnung des Gewichtes und des Alters eines Kindes, insbesondere in einer medizinischen Notfallsituation (sogenannter pädiatrischen Notfallrechner).

Notfallsituationen bei Kindern sind für medizinisches Personal in vieler Hinsicht eine Herausforderung. Oft fehlt ihnen die notwendige Erfahrung mit Kindernotfallsituationen, insbesondere bzgl. den Medikamentendosierungen, der korrekten Auswahl der altersentsprechenden medizinischen Ausrüstung und der Kenntnis der akzeptablen Vitalparametern sowie der Schätzung von Gewicht und Alter bei Fehlen von entsprechenden Angaben.

Hierzu wurden sogenannte Kindernotfallbänder entwickelt, welche es erlauben, die Kinder in der Länge auszumessen und darauf basierend entsprechende gewichtsbezogene Medikamentendosierungen und eine altersbezogene Ausrüstungsauswahl erlauben. Ein Beispiel für ein entsprechendes pädiatrisches Notfallband ist im US Patent US20060137696A1 beschrieben. Im deutschsprachigen Bereich ist das sogenannte pädiatrische Notfalllineal bekannt (Gebrauchsmusterschutz Nr. 202009011884.3 (Deutsches Patent und Markenamt, D-80297 München).

Diese Notfallbänder erlauben es dem medizinischen Personal bei Fehlen von Gewichts- und Altersangaben schnell die nötige Notfallmedikation in der richtigen Dosierung bereitzustellen bzw. zu applizieren und das medizinische Material wie Beatmungsmaske, Endotrachealer Tubus in der korrekten Größe auszuwählen bzw. anzuwenden.

Die Nachteile der Systeme sind jedoch, dass sie bei der längenbasierten Gewichtsschätzung das Geschlecht, den Habitus und die Ethnizität des Kindes/Patienten nicht berücksichtigen. Zwar erwägen die Autoren dieser Notfallbänder entsprechend dem Habitus den Patienten in eine nächst tiefere bzw. eine nächst höhere Gewichtskategorie einzuordnen. Dies mag für die Dosierung von Medikamenten geeignet sein, die Auswahl von medizinischem Equipment richtet sich aber nicht nach dem Habitus, sondern nach dem Alter des Patienten, welche vor allem durch Länge geschätzt wird bzw. im Atemwegsbereich v.a. mit der Körperlänge des Patienten korreliert. Des Weiteren wurde festgestellt, dass dadurch keine bessere Gewichtsschätzung erzielt werden kann. Ein weiterer wesentlicher Nachteil dieser Bänder ist die Kategorisierung in eine beschränkte Anzahl von Längengruppen, was die Gewichts- und Altersschätzung wiederum ungenau macht. Ebenso wird auf den Notfallbändern eine große Anzahl von Informationen in ein relativ kleines Feld komprimiert. Schließlich decken diese Bänder nur einen gewissen Körperlängenbereich wie z. Bsp. 50 - 140 cm ab. Wird bei einem Notfallpatienten durch Verwandten- oder Fremdangaben das korrekte Alter bekannt, kann es auf dem Notfallband nicht angewandt werden, sodass mindestens die Materialauswahl entsprechend angepasst werden könnte.

Daher liegt der vorliegenden Erfindung die Aufgabe zugrunde, eine Einrichtung bereitzustellen, die im Hinblick auf die oben genannten Probleme verbessert ist.

Diese Aufgabe wird durch eine Einrichtung mit den Merkmalen des Anspruchs 1 gelöst. Vorteilhafte Ausgestaltungen der Erfindung sind in den Unteransprüchen angegeben und werden nachfolgend beschrieben.

Gemäß Anspruch 1 ist danach eine Einrichtung zur Berechnung des Gewichtes und des Alters eines Kindes vorgesehen, insbesondere in einer medizinischen Notfallsituation, wobei die Einrichtung aufweist:
- ein Gehäuse;
- eine Längenmesseinheit, die zum Messen der Länge des Körpers des Kindes ausgebildet ist;
- eine im Gehäuse angeordnete elektronische Recheneinheit, die dazu konfiguriert ist unter Verwendung der gemessenen Länge eine automatische (und insbesondere kontinuierliche) Berechnung des Gewichtes und/oder des Alters des Kindes vorzunehmen.

Unter einer kontinuierlichen Längenmessung wird im Rahmen der vorliegenden Erfindung die Möglichkeit verstanden, die exakte Länge (bis auf etwaige Messungenauigkeiten) des Patienten mit der Längenmesseinheit zu bestimmen. Bisherige Notfallbänder sind kategorisiert, d.h. in bestimmte Längensegmente, z.B. 50 bis 60 cm, 60 bis 70 cm usw. unterteilt. Bei der vorliegenden Erfindung kann jedoch insbesondere für jeden gemessenen (exakten) Längenwert ein zugeordnetes Gewicht (und entsprechend die Dosierung der Medikamente) und das Alter (entsprechend dann die Auswahl der Atemwegsdevices) berechnet werden.

Grundsätzlich besteht bei allen Ausführungsformen der Erfindung bevorzugt die Möglichkeit, dass die gemessene Länge nur manuell eingebbar ist oder zusätzlich manuell eingebbar ist (fail safe). So kann z.B. auch für den Fall, dass die Längenmesseinheit defekt ist, die Länge auf eine alternative Weise bestimmt werden und dann eingegeben werden. Die Einrichtung weist also vorzugsweise ein Eingabemittel zum manuellen Eingeben der gemessenen Länge des Patienten auf. Ein solches Eingabemittel wird bevorzugt auch bei Ausführungsformen der Erfindung verwendet, bei denen keine integrierte Längenmesseinheit vorhanden ist, sondern eine separate Längenmesseinheit (z.B. ein herkömmliches analoges Maßband).

Weiterhin ist die Einrichtung, insbesondere die Recheneinheit, bevorzugt dazu konfiguriert, dass Gewicht und Alter insbesondere unter Berücksichtigung des Geschlechtes, des Habitus (auch als Körperhabitus bezeichnet) und der Ethnizität des kindlichen Patienten zu berechnen.

Weiterhin ist die Einrichtung, insbesondere die Recheneinheit, bevorzugt dazu konfiguriert, die Körperoberfläche (KOF), den Body-Mass-Index (BMI) und zusätzlich zum errechneten tatsächlichen Körpergewicht (TKG), das Ideal-Körpergewicht (IKG) und das Lean-Körpergewicht (LKG; Lean-Body-Weight) zu berechnen, und zwar insbesondere unter Berücksichtigung des Geschlechtes, des Habitus (auch als Körperhabitus bezeichnet) und insbesondere der Ethnizität des kindlichen Patienten.

Hierdurch ist es mit Vorteil möglich das Körpergewicht des Kindes besser abzuschätzen und damit Medikamente genauer zu dosieren. So können die Medikamentendosierungen je pharmakokinetischen und -dynamischen Medikamenteneigenschaften, basierend auf dem tatsächlichen Gewicht, dem Idealgewicht oder dem Lean-Körpergewicht berechnet werden. Die Längenmesseinheit kann insbesondere ein kontinuierliches Maßband aufweisen (siehe auch unten), das es erlaubt, die exakte Länge des Kindes zu erfassen und in eine Recheneinheit eines elektronischen Mediums zur weiteren Prozesssierung (Modifikation nach Habitus, Geschlecht, uns insbesondere Ethnizität) einzutippen und damit zur Berechnung von Alter, Körpergewicht, sowie ggf. KOF, BMI, TKG, IKG und LGK einzusetzen. Die elektronische Verfügbarkeit der Längenangabe erlaubt sogar, diese Information direkt, d. h., ohne Eintippen in ein System, in die Recheneinheit der Einrichtung zur weiteren Prozesssierung (Modifikation nach Habitus, Geschlecht, Ethnizität) zu übertragen und damit zur Berechnung von Alter, Körpergewicht, sowie ggf. KOF, BMI, TKG, IKG und LGK einzusetzen.

Gemäß einer bevorzugten Ausführungsform der erfindungsgemäßen Einrichtung ist vorgesehen, dass die Längenmesseinheit in das Gehäuse integriert ist. Dies bedeutet insbesondere, dass zumindest eine Komponente der Längenmesseinheit insbesondere sämtliche Komponenten der Längenmesseinheit dauerhaft am Gehäuse festgelegt sind. Sofern zur Längenmessung ein Laser oder eine Ultraschallquelle verwendet wird, kann zum Beispiel am Gehäuse ein abnehmbarer Reflektor vorgesehen sein, der zur Längenmessung vom Gehäuse abgenommen wird und nach erfolgter Längenmessung wieder am Gehäuse festgelegt werden kann.

Gemäß einer alternativen Ausführungsform ist vorgesehen, dass die Längenmesseinheit separat ausgebildet ist, z.B. in Form eines herkömmlichen Maßbandes. Ein solches separates Maßband kann zur analogen oder elektronischen Längenmessung ausgebildet sein. Die separate Längenmesseinheit kann auch eine andere beliebige Längenmesseinheit zum Messen der Länge des Kindes sein.

Für den Fall, dass ein (z.B. elektronisches oder analoges/mechanisches) ausziehbares Maßband verwendet wird, ist insbesondere vorgesehen, dass das Gehäuse ein Sichtfenster aufweist, das es erlaubt, die gemessene Länge (ausgezogenes Maßband), nebst der elektronischen Erfassung, auch visuell zu erfassen.

Weiterhin ist gemäß einer bevorzugten Ausführungsform der erfindungsgemäßen Einrichtung vorgesehen, dass die Einrichtung ein Eingabemittel zum Eingeben des Geschlechts aufweist.

Weiterhin ist gemäß einer bevorzugten Ausführungsform der erfindungsgemäßen Einrichtung vorgesehen, dass die Einrichtung ein Eingabemittel zum Eingeben des Habitus aufweist.

Weiterhin ist gemäß einer bevorzugten Ausführungsform der erfindungsgemäßen Einrichtung vorgesehen, dass die Einrichtung ein Eingabemittel zum Eingeben der Ethnizität des Kindes aufweist,

Bevorzugt ist weiterhin die elektronische Recheneinheit dazu konfiguriert, einen oder mehrere dieser Informationen (das Geschlecht, den Habitus, die Ethnizität) bei der Berechnung des Gewichtes zu verwenden.

Unter einem Habitus wird im Rahmen der vorliegenden Erfindung insbesondere ein Ernährungszustand des Patienten verstanden, der vom Anwender zu schätzen ist. Der Habitus kann z.B. in feste Kategorien wie z.B. "schlank", "normal" oder "adipös" unterteilt sein. Der Patient ist dann in zumindest eine der Kategorien einzuordnen. Es kann ggf. auch eine feinere Abstufung des Habitus vorgesehen sein.

Die Einrichtung kann grundsätzlich gemäß in einer Ausführungsform dazu ausgebildet sein, die Habitus kann als Zeichen (z.B. Plus und Minus), als Symbol, als Zeichnung, oder als Fotos zur Auswahl anzuzeigen, zusätzlich können die Zeichnungen oder Fotos altersspezifisch und geschlechtsspezifisch ausgebildet sein.

Die Einrichtung, insbesondere die elektronische Recheneinheit (oder der separate Rechner, siehe unten) ist bevorzugt dazu konfiguriert, anhand der gemessenen Länge das Alter des Kindes sowie das Gewicht zu berechnen. Hierzu verwendet die Einrichtung bevorzugt einen entsprechenden (durch die Recheneinheit bzw. den Rechner ausgeführten) Algorithmus.

Bei Errechnung des Alters wird vom Algorithmus bevorzugt die 50te Längen-Alter-Perzentile verwendet und das der gemessenen Länge entsprechende Alter als Ergebnis der Berechnung verwendet. Bevorzugt wird die dem eingegebenen Geschlecht sowie insbesondere der eingegebenen Ethnizität entsprechende Längen-Alter-Perzentile verwendet.

Bei der Gewichtsberechnung wird hingegen bevorzugt (abhängig vom eingegebenen Habitus) die 50te Längen-Gewichts-Perzentile bei einem normalen Habitus verwendet, sowie eine niedrigere bzw. höhere Perzentile bei schlankem bzw. adipösen Habitus. Das der gemessenen Länge entsprechende Gewicht wird als Ergebnis der Berechnung verwendet. Bevorzugt wird die dem eingegebenen Habitus, Geschlecht sowie insbesondere der eingegebenen Ethnizität entsprechende Längen-Gewichts-Perzentile verwendet.

Weiterhin ist die Einrichtung bzw. der Algorithmus vorzugsweise dazu ausgebildet, die KOF (Körperoberfläche), den BMI sowie das tatsächliche Körpergewicht (TKG), das Ideal-Körpergewicht (IKG) und das Lean-Körpergewicht (LKG; Lean-Body-Weight) des Kindes zu berechnen. Hierzu verwendet der Algorithmus jeweils vorzugsweise eine aus dem Stand der Technik bekannte, validierte Formel, die als Input das berechnete Gewicht und die gemessene Länge verwendet.

Weiterhin ist gemäß einer bevorzugten Ausführungsform der erfindungsgemäßen Einrichtung vorgesehen, dass die Einrichtung ein Eingabemittel aufweist, das zum Quittieren der gemessenen Länge durch den Anwender ausgebildet ist. Hierbei kann es sich zum Beispiel um ein mittels eines Fingers betätigbares Betätigungselement, insbesondere in Form einer Taste, handeln.

Hierdurch kann mit Vorteil der Zeitpunkt, zu dem die Längenmesseinheit die ermittelte Länge an die Recheneinheit übergibt bzw. dieser zur Verfügung stellt, durch den Verwender der Einrichtung bestimmt werden, und zwar durch Quittieren der gemessenen Länge mittels des entsprechenden Eingabemittels.

Weiterhin ist gemäß einer bevorzugten Ausführungsform der erfindungsgemäßen Einrichtung vorgesehen, dass die Einrichtung ein Eingabemittel zum Korrigieren des berechneten Gewichtes und/oder des berechneten Alters aufweist. Hierbei kann es sich zum Beispiel ebenfalls jeweils um ein mittels eines Fingers betätigbares Betätigungselement, insbesondere in Form je einer Taste, handeln.

Weiterhin ist gemäß einer bevorzugten Ausführungsform der erfindungsgemäßen Einrichtung vorgesehen, dass die Einrichtung ein Bestätigungsmittel für die eingegebene Länge oder übernommene Länge aufweist, des berechneten Gewichtes und/oder des berechneten Alters aufweist. Hierbei kann es sich zum Beispiel ebenfalls jeweils um ein mittels eines Fingers betätigtes Betätigungselement, insbesondere in Form je einer Taste oder eines Feldes auf dem Display handeln. Die Betätigung des Bestätigungsmittels löst dann die Berechnung des Gewichtes und des Alters (sowie ggf. der KOF, des BMI, des TKG, IKG und LKG) aus.

Weiterhin ist gemäß einer bevorzugten Ausführungsform der erfindungsgemäßen Einrichtung vorgesehen, dass die Einrichtung ein Anzeigemittel aufweist, insbesondere zum Anzeigen der gemessenen Länge sowie des berechneten Gewichtes und/oder des berechneten Alters (sowie ggf. der KOF, des BMI, des TKG, IKG und LKG) aufweist. Bevorzugt handelt es sich bei dem Anzeigemittel um ein optisches Anzeigemittel in Form eines Bildschirms (Display), z.B. in Form eines LCD, TFT-Displays, oder OLED-Displays. Andere optische Anzeigemittel sind auch denkbar.

Weiterhin ist gemäß einer bevorzugten Ausführungsform der erfindungsgemäßen Einrichtung vorgesehen, dass das Anzeigemittel sowie die Eingabemittel am Gehäuse angeordnet sind. Bei der Einrichtung handelt es sich insbesondere in diesem Fall um eine Stand-Alone-Device die sämtliche Komponenten der Einrichtung in sich vereinigt, wobei insbesondere die besagte Längenmesseinheit in das Gehäuse der Einrichtung integriert ist (siehe auch unten).

Es ist aber gemäß einer Ausführungsform auch möglich, dass das Anzeigemittel ein separates Gerät ist, das mit dem Gehäuse, dass die Recheneinheit aufnimmt, über eine geeignete Datenübertragungsverbindung verbindbar ist (z.B. WiFi, Bluethooth, Kabel).

Weiterhin ist gemäß einer bevorzugten Ausführungsform der erfindungsgemäßen Einrichtung vorgesehen, dass das Anzeigemittel ein am Gehäuse angeordneter (oder getrennt vom Gehäuse ausgebildeter) Touchscreen ist. Dies erlaubt es auch einzelne oder alle Eingabemittel mittels des Touchscreens zu realisieren. D.h. eine beliebige Auswahl der hierin beschriebenen Eingabemittel kann durch den Touchscreen realisiert werden. Die restlichen vorhandenen Eingabemittel können separat dazu vorgesehen werden (z.B. am Gehäuse).

Bei der erfindungsgemäßen Einrichtung kann es sich insbesondere um einen Tablet-PC handeln, in den dann bevorzugt die Längenmesseinheit sowie Recheneinheit integriert ist (es muss insbesondere keine gesonderte Recheneinheit vorhanden sein, da auch der Prozessor des Tablet-PCs/Smartphones verwendet werden kann) Bei einem solchen Tablet-PC/Smartphone handelt es sich um einen Computer bzw. Mobiltelefon mit einem Touchscreen als Anzeige- und Eingabemittel, wobei dieser Computer insbesondere keine ausklappbare bzw. gelenkig mit einem Bildschirm verbundene Tastatur aufweist.

Für den Fall dass die erfindungsgemäße Einrichtung ein Anzeigemittel in Form eines Touchscreens aufweist bzw. als Tablet PC mit einer integrierten Längenmesseinheit ausgebildet ist, kann gemäß einer Ausführungsform der Erfindung vorgesehen sein, dass die Eingabemittel zum Eingeben des Geschlechts, des Habitus und der Ethnizität des Kindes sowie zum Quittieren der Messung separat zum Touchscreen ausgebildete Eingabemittel sind, die zum Beispiel benachbart zum Touchscreen am Gehäuse angeordnet sein können. Die erfindungsgemäße Einrichtung kann in diesem Fall also die Gestalt eines herkömmlichen Tablet PCs aufweisen, der um die besagten separaten Eingabemittel erweitert ist und eine integrierte Längenmesseinheit aufweist (siehe oben).

Weiterhin kann insbesondere das besagte Eingabemittel zum Korrigieren des Gewichtes oder Alters (siehe oben) durch den Touchscreen gebildet sein (z.B. als auf dem Touchscreen auswählbare bzw. betätigbare Icons, die z.B. durch einen entsprechenden, durch die Recheneinheit ausgeführten Programmcode erzeugt sind).

Schließlich können die besagten Eingabemittel sämtlich durch den Touchscreen gebildet sein bzw. bereitgestellt werden (z.B. als auf dem Touchscreen auswählbare bzw. betätigbare Icons, die z.B. durch einen entsprechenden durch die Recheneinheit ausgeführten Programmcode erzeugt sind).

Allgemein bzw. insbesondere bei dieser Ausführungsform können die Habitus-Bilder "mager"/"normal"/übergewichtig" entsprechend dem Alter für Neugeborene, Säuglinge, Kleinkinder, Vorschulinder, Schulkinder, Adoleszente und junge Erwachsene, ggf. unterschiedlich für männlich und weiblich auf dem Anzeigemittel erscheinen, um es dem Rettungspersonal etwas illustrativer zu machen, wie ein altersentsprechend normaler oder eben nicht normaler Habitus aussieht.

Weiterhin kann die Einrichtung gemäß einer alternativen Ausführungsform der Erfindung einen separaten Rechner aufweisen, bei dem es sich insbesondere um einen Tablet PC oder ein Smartphone handeln kann, wobei nunmehr die elektronische Recheneinheit der Einrichtung bevorzugt dazu ausgebildet ist, eine, mehrere oder alle der folgenden Informationen auf den separaten Rechner zu übertragen: die gemessene Länge, das berechnete Gewicht, das berechnete Alter, das eingegebene Geschlecht des Kindes, den eingegebenen Habitus des Kindes, die eingegebene Ethnizität des Kindes.

Diesbezüglich kann die Einrichtung gemäß einer Ausführungsform dazu konfiguriert sein, die besagten Informationen über eine Kabelverbindung, eine Verbindung (insbesondere Steckverbindung oder sonstige Kontaktverbindung) oder eine drahtlose Verbindung (z.B. WiFi oder Bluetooth) auf den separaten Rechner bzw. Tablet-PC zu übertragen.

Weiterhin ist gemäß einer bevorzugten Ausführungsform der erfindungsgemäßen Einrichtung vorgesehen, dass das Gehäuse ein Mittel zum vorzugsweise lösbaren Festlegen des separaten Rechners am Gehäuse aufweist, wobei das Gehäuse insbesondere eine Ausnehmung oder einen Rahmen zum Aufnehmen des separaten Rechners aufweisen kann, wobei das Gehäuse insbesondere zum lösbaren Festlegen des separaten Rechners bzw. Tablet-PCs in der Ausnehmung eingerichtet und vorgesehen ist.

Die besagte Verbindung (insbesondere Steckverbindung) kann hierbei eine erste, am Gehäuse vorgesehene Komponente sowie eine zweite, am Rechner bzw. Tablet-PC vorgesehene Komponente aufweisen, die miteinander in Eingriff bringbar sind und/oder miteinander kontaktier sind, so dass eine Datenverbindung zwischen dem separaten Rechner (z.B. Tablet-PC) und der Recheneinheit hergestellt wird, wenn die beiden Komponenten bestimmungsgemäß miteinander in Eingriff stehen bzw. einander kontaktieren. Hierbei sind die beiden Komponenten der Steckverbindung so konfiguriert, dass der besagte Eingriff bzw. Kontakt hergestellt wird, indem der separate Rechner bestimmungsgemäß am Gehäuse festgelegt und/oder in der besagten Ausnehmung oder dem Rahmen angeordnet wird. Vorzugsweise ist der separate Rechner formschlüssig in der Ausnehmung oder dem Rahmen anordenbar.

Weiterhin ist gemäß einer Ausführungsform der Erfindung vorgesehen, dass die Eingabemittel zum Eingeben des Geschlechts, des Habitus und der Ethnizität des Kindes sowie zum Quittieren der Messung der Länge separat zum Touchscreen bzw. Anzeigemittel ausgebildete Eingabemittel sind, die z.B. benachbart zum Touchscreen/Anzeigemittel am Gehäuse angeordnet sind.

Weiterhin ist gemäß einer Ausführungsform der erfindungsgemäßen Einrichtung vorgesehen, dass das Eingabemittel zum Korrigieren des Gewichtes oder Alters (siehe oben) durch den Touchscreen gebildet ist (z.B. als auf dem Touchscreen auswählbare bzw. betätigbare Icons, die z.B. durch einen entsprechenden durch die Recheneinheit ausgeführten Programmcode erzeugt sind).

Weiterhin ist gemäß einer Ausführungsform der erfindungsgemäßen Einrichtung vorgesehen, dass die Eingabemittel sämtlich durch den Touchscreen gebildet sind bzw. bereitgestellt werden (z.B. als auf dem Touchscreen auswählbare bzw. betätigbare Icons, die z.B. durch einen entsprechenden durch die Recheneinheit ausgeführten Programmcode erzeugt sind).

Weiterhin ist gemäß einer bevorzugten Ausführungsform der Erfindung vorgesehen, dass die Einrichtung, insbesondere die Recheneinheit oder der separate Rechner, dazu konfiguriert ist, eine Dosierungsberechnung eines dem Kind zu verabreichenden Medikaments und/oder eine Materialberechnung durchzuführen und die berechnete Dosis sowie das berechnete Material (insbesondere mittels des Anzeigemittels oder Touchscreens) anzuzeigen. Bei den besagten Materialien, die zu berechnen (z.B. Berechnung einer Größe des Materials) bzw. auszuwählen sind, kann es sich insbesondere um einen oder mehrere der folgenden Gegenstände handeln:
- ein endotrachealer Tubus
- eine Larynxmaske
- eine Beatmungsgesichtsmaske
- ein oro-pharyngeal Tubus
- ein naso-Pharyngeal Tubus
- ein Laryngoskopie-Spatel (Miller und Maclntosh)
- eine intraossäre Nadel
- ein Beatmungsbeutel
- Führungsstab für endotrachealer Tubus
- Thoraxdrainage
- Absaugkatheter
- Magensonde
- Blasenkatheter
- I-Gel Maske
- Larynxtubus
- Blutdruckmanschette

Weiterhin ist die Einrichtung, insbesondere die Recheneinheit oder der separate Rechner, ferner bevorzugt dazu konfiguriert, für die Dosierungsberechnung oder die Materialberechnung eine, mehrere oder sämtliche der folgenden Informationen zu verwenden: die gemessene Länge, das berechneten Gewicht (insbesondere TKG, IKG und/oder LKG), das berechnete Alter, das Geschlecht des Kindes, der Habitus des Kindes, die Ethnizität des Kindes.

Weiterhin ist gemäß einer bevorzugten Ausführungsform der Erfindung vorgesehen, dass die Längenmesseinheit ein ausziehbares Maßband aufweist, das aus dem Gehäuse ausziehbar ist, wobei die Längenmesseinheit dazu konfiguriert ist, die aus dem Gehäuse ausgezogene Länge des Maßbandes automatisch zu bestimmen (sogenanntes elektronisches Maßband) und an die Recheneinheit oder den separaten Rechner zu übermitteln bzw. der Recheneinheit oder dem separaten Rechner zur Verfügung zu stellen. Vorzugsweise ist das Maßband so ausgebildet, dass es in einer ausgezogenen Position arretierbar und/oder selbsttätig wieder in das Gehäuse einziehbar ist (z.B. durch ein Federmittel, ggf. nach Lösen einer erfolgten Arretierung).

Anstelle eines elektronischen Maßbandes kann auch ein analoges Maßband verwendet werden. In diesem Fall kann z.B. am Gehäuse ein Sichtfenster vorgesehen sein, in dem die ausgezogene Länge ablesbar ist. Die Einrichtung weist in diesem Fall ein Eingabemittel zum manuellen Eingeben der analog gemessenen Länge auf, z.B. in Form eines separaten, am Gehäuse ausgebildeten Eingabemittels oder in Form eines durch das Anzeigemittel (Touchscreen) bereitgestellten Eingabemittels. Alternativ zu einem Maßband kann die Längenmesseinheit zum Messen der Länge des Kindes einen Laser oder eine Ultraschallquelle sowie einen lösbar am Gehäuse festlegbaren Reflektor aufweisen. Der Reflektor wird dann an einem Körperende (z.B. den Füßen) und die Längenmesseinheit bzw. das Gehäuse am anderen Körperende (z.B. am Kopf) positioniert und dient zum Reflektieren des vom Laser erzeugten Laserlichts bzw. des von der Ultraschallquelle erzeugten Ultraschalls. Die Längenmesseinheit ist wiederum dazu ausgebildet die solchermaßen gemessene Länge an die Recheneinheit oder den separaten Rechner zu übermitteln bzw. der Recheneinheit oder dem separaten Rechner zur Verfügung zu stellen. Nach der Messung kann der Reflektor wieder am Gehäuse festgelegt werden (z.B. mittels einer Rastverbindung oder einer sonstigen Verbindung).

Weiterhin ist gemäß einer bevorzugten Ausführungsform der Erfindung vorgesehen, dass die Einrichtung, insbesondere die Recheneinheit und/oder der separate Rechner, dazu ausgebildet ist, Patientenvitaldaten (z.B. einen nicht-invasiven Blutdruck, invasive Drücke, transkutane arterielle und regionale Sauerstoffsättigungen, Atemgaswerte, ein Elektrokardiogramm, eine Temperatur, eine Schlaftiefe, eine neuromuskuläre Relaxation, Spirometrie) sowie Ultraschallbilder mittels konnektierbaren Sensoren bzw. Sonden zu messen und mittels des Anzeigemittels (z.B. Touchscreen) die Patientenvitaldaten bzw. Ultraschallbilder anzuzeigen.

Hierbei ist bevorzugt vorgesehen, dass die Einrichtung Verbindungsmittel (z.B. eine Kabelverbindung oder eine drahtlose Verbindung) zum Herstellen einer Datenübertragungsverbindung zwischen zumindest einem Sensor bzw. Sonden zum Messen von Patientenvitaldaten (z.B. EKG, Herzfrequenz, Sauerstoffsättigung transkutan, Blutdruckmessung, Temperatur, ETCO₂, NIRS, BIS) bzw. Darstellen von Körperstrukturen und der Einrichtung aufweist.

Grundsätzlich ist es denkbar, dass die Einrichtung gemäß einer Ausführungsform keine in das Gehäuse integrierte Längenmesseinheit, sondern eine separate Längenmesseinheit aufweist, wobei die Länge des Körpers des Patienten mittels der Längenmesseinheit bestimmbar ist und nunmehr die Einrichtung ein am Gehäuse vorgesehenes Eingabemittel zum manuellen Eingeben der gemessenen Länge aufweist, wobei wiederum dieses Eingabemittel durch den oben erwähnten Touchscreen bereitgestellt bzw. gebildet sein kann, oder wobei alternativ dieses Eingabemittel ein separates, am Gehäuse der Einrichtung vorgesehenes Eingabemittel ist, dass zum Beispiel benachbart zum Anzeigemittel bzw. Touchscreen der Einrichtung am Gehäuse angeordnet ist.

Weiterhin ist die erfindungsgemäße Einrichtung gemäß einer Ausführungsform dazu ausgebildet sein, ein Alarmsignal, insbesondere in Form eines visuellen Alarms, eines akustischen Alarms, und/oder eines haptischen Alarms (z.B. Vibration) zu erzeugen.

Weiterhin ist die erfindungsgemäße Einrichtung gemäß einer Ausführungsform dazu ausgebildet, eine oder mehrere Audiodateien abzuspielen, die Informationen, enthalten, insbesondere über am Patienten vorzunehmende Maßnahmen.

Weiterhin weist die erfindungsgemäße Einrichtung gemäß einer Ausführungsform für das Eingabemittel zur Eingabe des Habitus altersentsprechende und/oder geschlechtsspezifische grafische Darstellungen (insbesondere in Form von Zeichnungen, Piktogrammen, Fotos) auf. Hierdurch kann die Habitus-Eingabe schnell und intuitiv erfolgen, was insbesondere im Notfall von großer Bedeutung ist.

Weiterhin weist die erfindungsgemäße Einrichtung gemäß einer Ausführungsform für das Eingabemittel zur Eingabe der Ethnizität ethnizitätsspezifische grafische Darstellungen (insbesondere in Form von Zeichnungen, Piktogrammen, Fotos) auf. Hierdurch kann die Ethnizitäts-Eingabe ebenfalls schnell und intuitiv erfolgen, was insbesondere im Notfall von großer Bedeutung ist.

Weiterhin ist die erfindungsgemäße Einrichtung gemäß einer Ausführungsform dazu ausgebildet einen Einsatz, insbesondere Notfalleinsatz, in Form eines Filmes (insbesondere mit Tonspur) oder in Form einer Tonspur aufzuzeichnen und eine entsprechende Datei zu speichern.

Weiterhin ist die erfindungsgemäße Einrichtung gemäß einer Ausführungsform dazu ausgebildet, den Inhalt des Anzeigemittels auf einen weiteren externen Monitor zu spiegeln (d.h. auch auf diesem Monitor auszugeben).

Weiterhin ist die erfindungsgemäße Einrichtung gemäß einer Ausführungsform dazu ausgebildet, bei einer Dosisberechnung eines Medikaments eine oder mehrere der folgenden Informationen zu verwenden: TKG, IKG, LGK, pharmakodynamische Medikamenteneigenschaften, pharmakokinetischen Medikamenteneigenschaften.

Die Erfindung sowie weitere Merkmale und Vorteile der Erfindung sollen nachfolgend anhand von Ausführungsbeispielen im Zusammenhang mit den Figuren erläutert werden. Es zeigen:

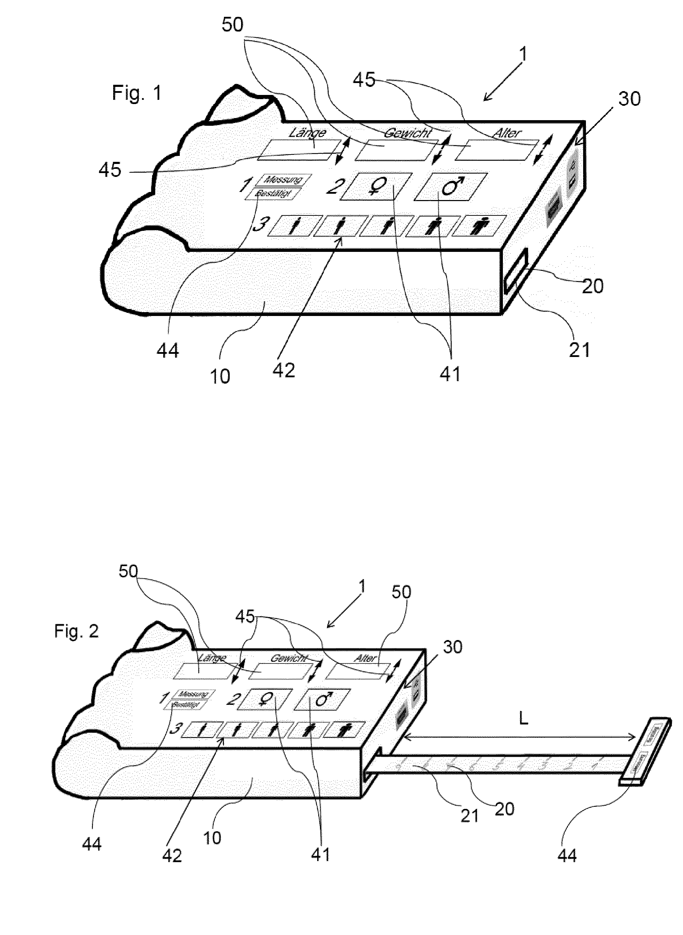

- Figur 1: eine perspektivische Ansicht eines Ausführungsbeispiels einer erfindungsgemäßen Einrichtung mit einer integrierten Längenmesseinheit (ohne ausgezogenes Maßband);
- Figur 2:: eine perspektivische Ansicht der Einrichtung gemäß Figur 1 mit ausgezogenem Maßband;

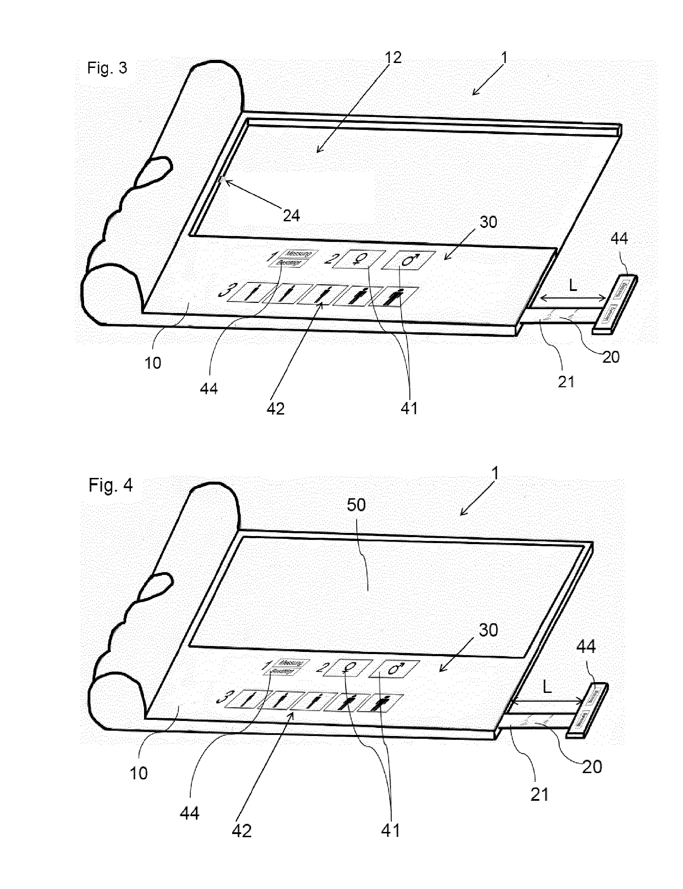

- Figur 3: eine perspektivische Ansicht einer erfindungsgemäßen Einrichtung mit einem Gehäuse, das eine Recheneinheit zum Bestimmen des Gewichts und des Alters des Patienten aus der Länge, des Geschlechtes und des Habitus des Patienten aufweist, wobei in das Gehäuse weiterhin eine Längenmesseinheit (mit Maßband) integriert ist, und wobei das Gehäuse eine Ausnehmung zum Andocken eines separaten Rechners, insbesondere Tablet-PC, aufweist;
- Figur 4: eine perspektivische Ansicht einer erfindungsgemäßen Einrichtung, in Form eines Tablet-PCs, wobei die Längenmesseinheit in das Gehäuse des Tablet-PCs integriert ist und weiterhin am Gehäuse separat zum Touchscreen vorgesehene Eingabemittel angeordnet sind, insbesondere zur Wahl des Geschlechts und des Habitus des kindlichen Patienten;

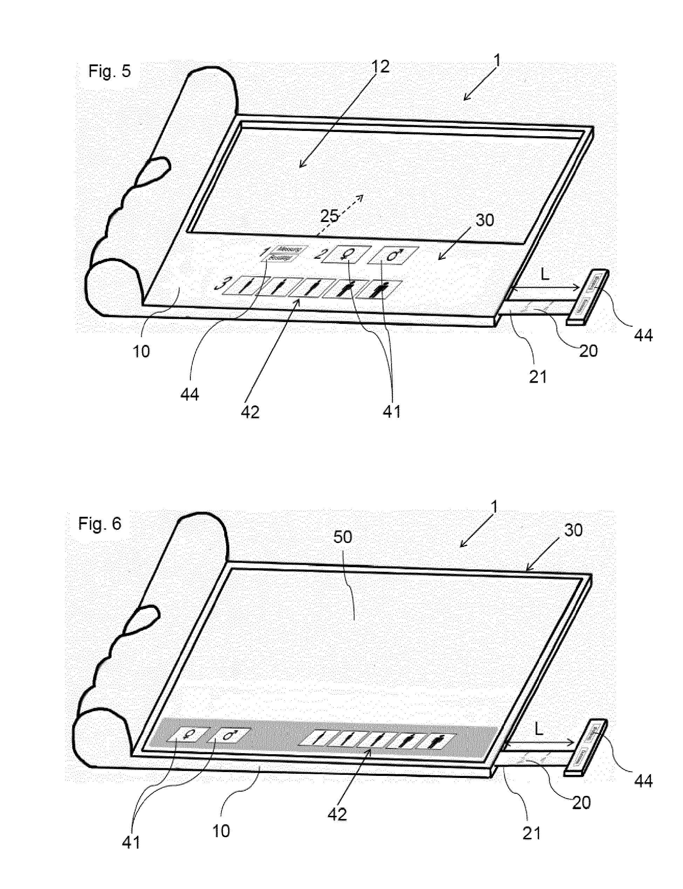

- Figur 5: eine perspektivische Ansicht einer Modifikation der in der Figur 3 gezeigten Einrichtung, wobei hier eine drahtlose Verbindung (z.B: WiFi oder Bluetooth) zwischen der im Gehäuse angeordneten Recheneinheit und dem separaten Rechner (z.B. Tablet-PC) hergestellt wird;
- Figur 6: eine perspektivische Ansicht einer Modifikation der in der Figur 4 gezeigten Ausführungsform, wobei hier die Eingabemittel zur Wahl des Geschlechts, des Habitus und der Ethnizität als Funktion des Touchscreens implementiert sind;

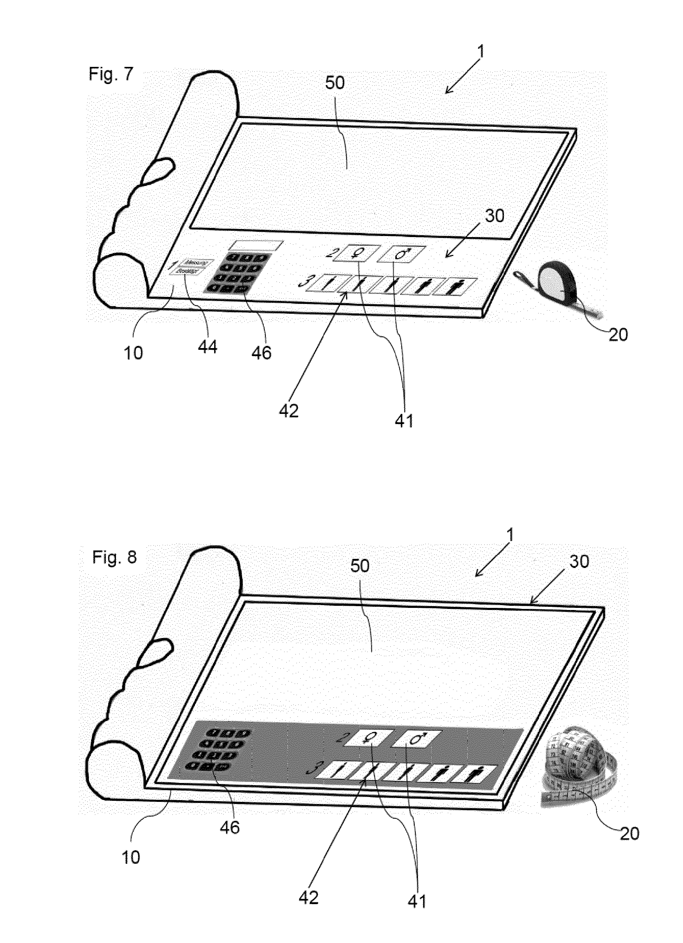

- Figur 7: eine perspektivische Ansicht einer erfindungsgemäßen Einrichtung mit einem Anzeigemittel (insbesondere Touchscreen) und separaten Eingabemitteln zur Wahl des Geschlechts, des Habitus und zur Eingabe von Zahlen, insbesondere der gemessenen Körperläge des Patienten, die hier mit einer separaten Längenmesseinheit messbar und mittels des entsprechenden Eingabemittels eingebbar ist;
- Figur 8: eine perspektivische Ansicht einer Modifikation der in der Figur 7 gezeigten Einrichtung, wobei hier die Eingabemittel zur Wahl des Geschlechts, des Habitus, der Ethnizität und zur Eingabe von Zahlen, insbesondere der gemessenen Körperläge des Patienten als Funktionen des Touchscreens implementiert sind;

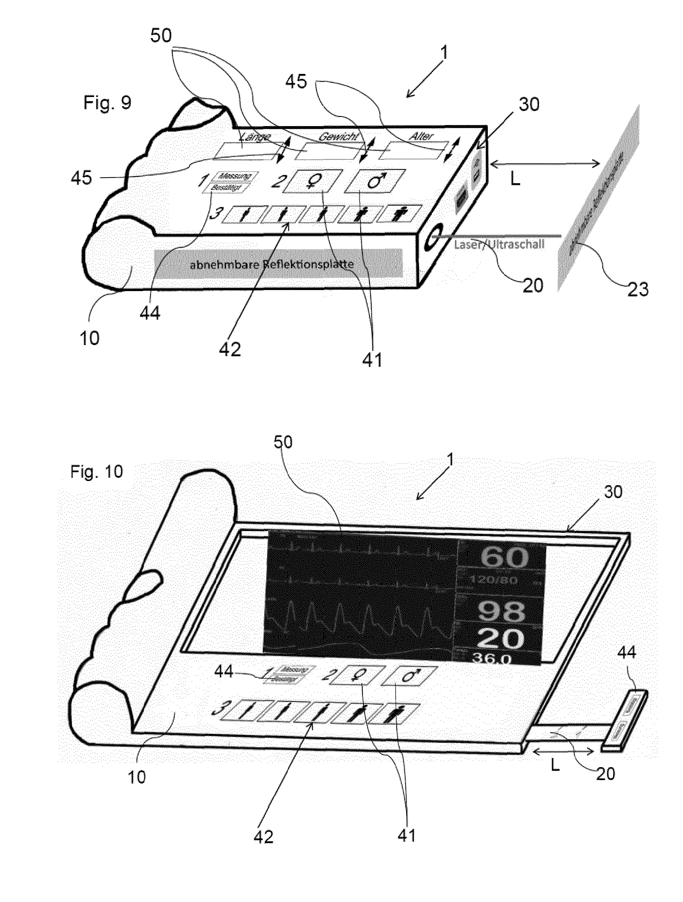

- Figur 9: eine perspektivische Ansicht einer Modifikation der in Figur 1 bzw. 2 gezeigten Einrichtung, wobei hier im Unterschied zu den Figuren 1 und 2 die Längenmesseinheit zur Bestimmung der Länge einen Laser oder eine Ultraschallquelle aufweist sowie einen in das Gehäuse integrierten und abnehmbaren Reflektor in Form einer Reflektionsplatte;
- Figur 10: eine erfindungsgemäße Einrichtung in Form eines Tablet-PCs, die zusätzlich zum Anzeigen und Überwachen von Vitaldaten (hier in Vollbilddarstellung) des Patienten ausgebildet ist;

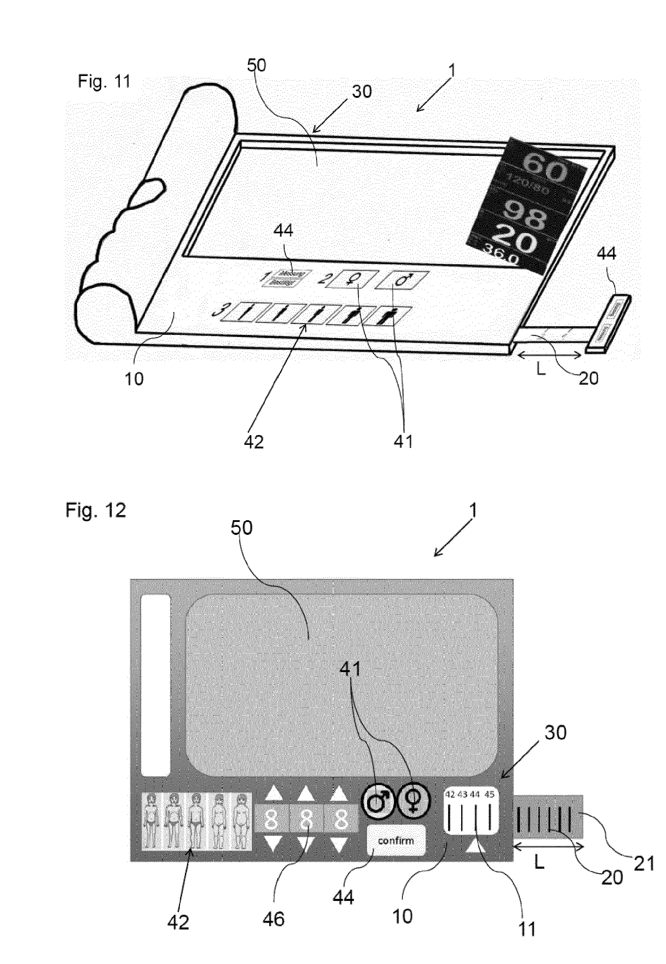

- Figur 11: die Einrichtung gemäß Figur 10, wobei hier das Vitaldatenmonitoring in einem Seitenfenster angezeigt wird (während einer Dosierungs-/Materialberechnung);
- Figur 12: eine Draufsicht auf eine erfindungsgemäße Einrichtung mit elektronischem und/oder mechanischem, ausziehbaren Maßband und Fenster zur Anzeige der gemessenen Länge auf dem Maßband. Die Einrichtung weist ein Gehäuse mit einem Anzeigemittel sowie separaten Eingabemitteln zum Eingeben von Informationen (Geschlecht und Habitus);

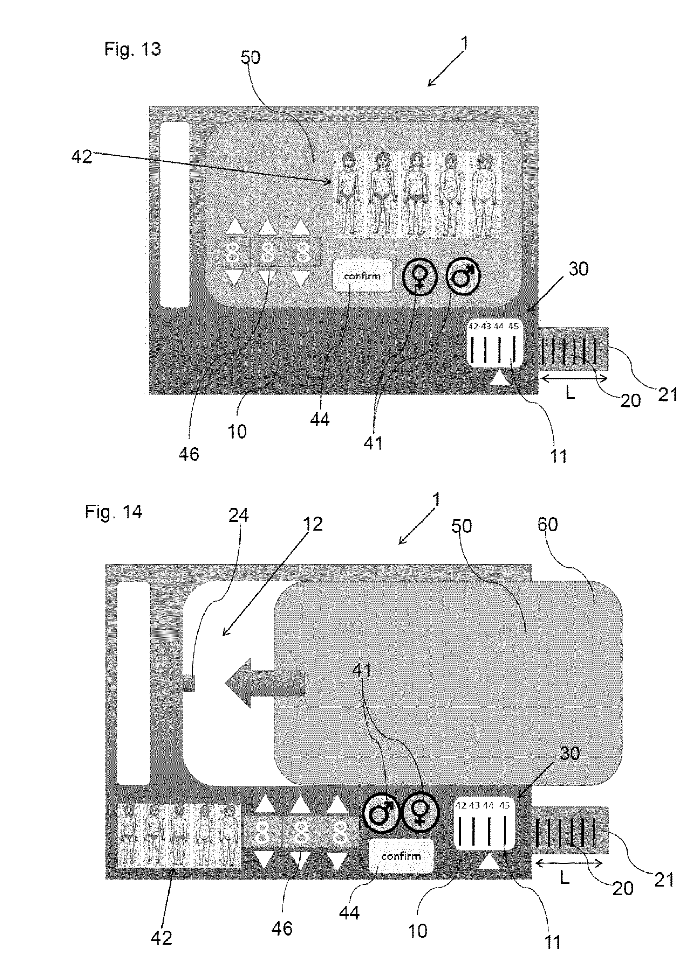

- Figur 13: eine Draufsicht auf eine Abwandlung der in der Figur 12 gezeigten Einrichtung mit elektronischem und/oder mechanischem Maßband und Fenster zur Anzeige der gemessenen Länge auf dem Band, wobei hier die Eingabemittel zur Wahl des Geschlechts, des Habitus, der Ethnizität und zur Eingabe von Zahlen, insbesondere der gemessenen Körperläge des Patienten als Funktionen des Touchscreens implementiert sind;
- Figur 14: eine Draufsicht auf eine weitere erfindungsgemäße Einrichtung mit elektronischem und/oder mechanischem Maßband und Fenster zur Anzeige der gemessenen Länge auf dem Band, wobei die Einrichtung ein Gehäuse mit einer Recheneinheit sowie mit Eingabemitteln zum Eingeben von Informationen (Geschlecht und Habitus) aufweist, und wobei das Gehäuse zum Aufnehmen eine separaten Rechners (insbesondere in Form eines Smartphones oder Tablet-PCs) ausgebildet ist, auf den die berechneten Werte übertragbar/anzeigbar sind;

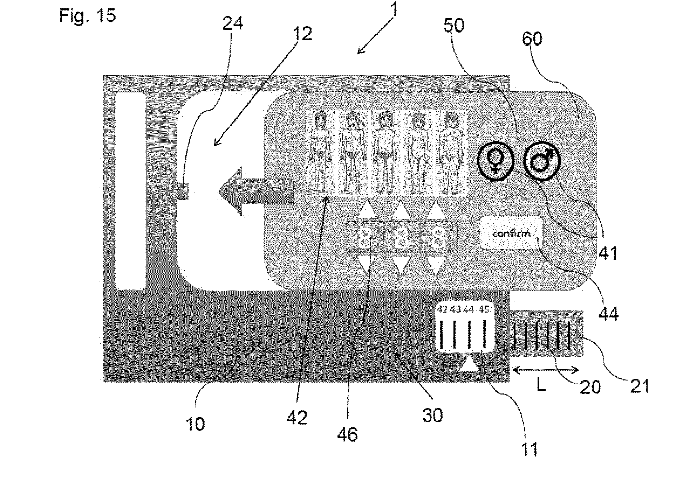

- Figur 15: eine Draufsicht auf eine Abwandlung der in der Figur 14 gezeigten Einrichtung mit integriertem elektronischem und/oder mechanischem Maßband und Fenster zur Anzeige der gemessenen Länge auf dem Band, wobei hier die Eingabemittel zur Wahl des Geschlechts, des Habitus, der Ethnizität und zur Eingabe von Zahlen, insbesondere der gemessenen Körperläge des Patienten als Funktionen des Touchscreens implementiert sind;

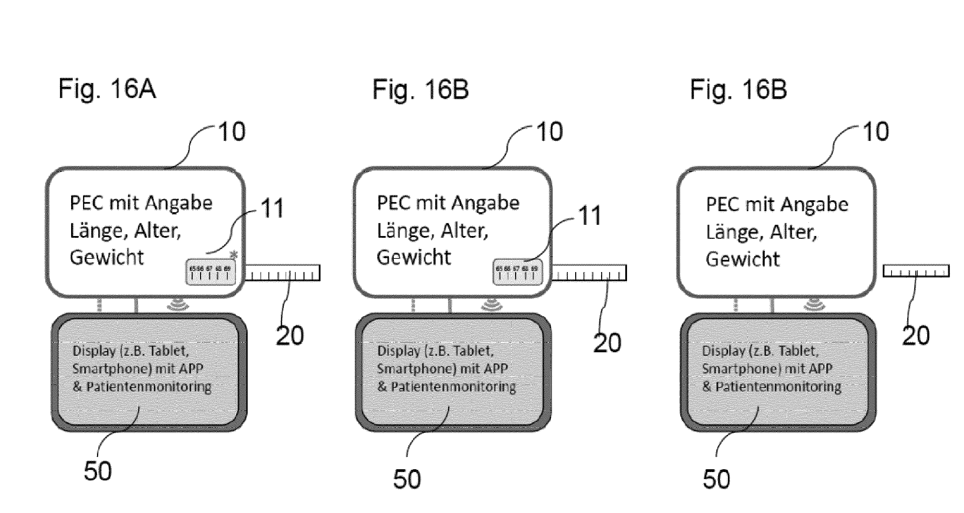

- Figuren 16A-16C: verdeutlichen noch einmal drei mögliche unterschiedliche Konfigurationen der erfindungsgemäßen Einrichtung;

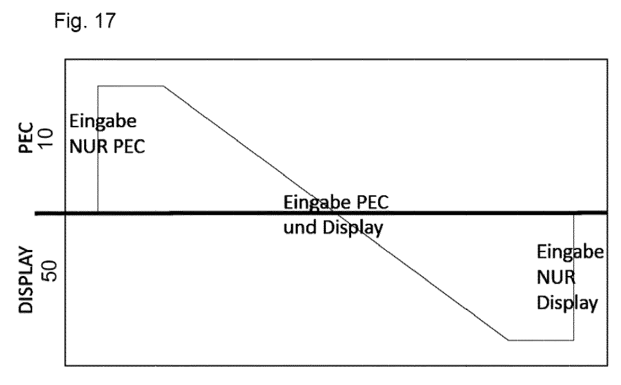

- Figur 17: zeigt im Zusammenhang mit Figuren 16A bis 16C mögliche Integrationsgrade der Eingabemittel.

Die erfindungsgemäße Einrichtung 1 soll im Folgenden zunächst hinsichtlich ihrer allgemeinen Merkmale und Eigenschaften beschrieben werden, wobei weiter unten zusätzlich, insbesondere anhand der Figuren, konkrete Ausführungsformen detailliert beschrieben werden.

Im Einsatzfall wird die erfindungsgemäße Einrichtung 1 z.B. mit der Seite, an der z.B. das Maßband ausgezogen werden kann, bündig an den Kopf des Patienten gelegt. Anschließend wird das Maßband parallel zum Patienten bis zum Fußende ausgezogen. Die Messung wird mittels Knopfdruck am Anfang des Maßbandes oder am Gehäuse des Notfallkalkulators selber bestätigt. Da es sich bei dem Maßband um ein elektronisches Maßband handelt, wird beim Ausziehen dessen ein digitales Signal entsprechend der erfassten Länge erstellt, welches elektrisch über ein Kabel oder eine Schnittstelle an die Recheneinheit weitergegeben und im Anzeigemittel (Display) in der Längenanzeige wiedergegeben und bestätigt (d.h. quittiert) wird. Anschließend werden Geschlecht, Habitus (drei bis mehr-stufig, z.B. von kachektisch bis adipös) und Ethnizität (z.B. Europa, Afrika, Asien, Nordamerika, Südamerika, Australien; weitere Aufgliederung nach Land möglich oder in der Grundeinstellung vorhanden) entsprechend der Anwender-Einschätzung gewählt. Basierend auf der gemessenen Länge und den bis zu drei Datensätzen (Geschlecht, Habitus und Ethnizität) adaptiert ein durch die Recheneinheit ausgeführter Algorithmus, der hierin auch als CLAWAR bezeichnet wird (für Continuous Length-based Algorithm for Weight & Age Rating), das Gewicht und das Alter des Patienten.

Die Daten "Gewicht (insbesondere TKG, LKG und/oder IKG), Alter, KOF und BMI" erlauben es, einem Dosis-Materialkalkulator Medikamentendosierungen (in "mg" und entsprechend der Medikamentenkonzentration sowie Flüssigkeitsmengen auch in "ml") zu berechnen und anzuzeigen.

Des Weiteren kann eine erfindungsgemäße Einrichtung eine Audio- bzw. Vorlesefunktion aufweisen. Hierbei ist die Einrichtung dazu konfiguriert (z.B. nach einem "Anklicken" eines entsprechenden Medikamentenpiktogramms oder eines Lautsprecher-Icons auf dem Touchscreen) eine Information vorzulesen bzw. eine entsprechende Audiodatei abzuspielen, z.B. Beispiel den Namen eines Medikamentes, eine Verdünnung eines Medikamentes und/oder eine Dosierung eines Medikamentes (z.B. in "mg" und "ml").

Das altersentsprechende und z.T. auch gewichtsentsprechende Atemwegsmaterial (Endotrachealer Tubus, Larynxmaske, Beatmungsmaske und Oro-Pharyngealtubus) sowie akzeptable Vitalparameter können ebenfalls angezeigt werden.

Auch hier kann die besagte Audiofunktion der Einrichtung ausgeführt werden, wobei z.B. nach einem "Anklicken" des Lautsprecher-Icons, Art und Größe des Materials und ggf. Vitalwertgrenzwerte vorgelesen werden. Zur Erleichterung in der Praxis werden die Materialien bevorzugt "farbcodiert" auf dem Anzeigemittel (Display, insbesondere Touchscreen) angezeigt. Entsprechend der Farbcodierung können vorgepackte Notfalltaschen mit dem entsprechenden, passenden Material vorgesehen werden.

Bevorzugt sind die Vitalgrenzwerte sind mit dem Vitaldatenmonitoring in Interaktion, wobei bei Unterschreitung und/oder Überschreitung insbesondere ein entsprechender akustischer und/oder optischer Alarm ausgelöst wird, wobei der optische Alarm z.B. ein Blinken des betreffenden bzw. gestörten Vitalgrenzwertes auf dem Anzeigemittel umfassen kann.

Die erfindungsgemäße Einrichtung kann ferner dazu konfiguriert sein, mittels einer Auslösetaste ausgelöste Foto- und/oder Filmaufnahmen (insbesondere mit Tonspur) zu erzeugen, um situative Bilder, Szenen und Krankheitsbilder aufzeichnen zu können.

Die erfindungsgemäße Einrichtung kann weiterhin dazu konfiguriert sein, einen Notfalleinsatz akustisch zu dokumentieren, insbesondere mit Zeitangabe, d.h. Gesprochenes wird dokumentiert.

Die erfindungsgemäße Einrichtung kann weiterhin dazu konfiguriert sein, eine gemessene Länge, eingegebene Daten, berechnete Daten, gemessene Vitaldaten, erhobene Ultraschallbilder/-sequenzen sowie Foto-, Film- und/oder Ton-Aufnahmen fallspezifisch zu speichern, wobei diese Daten insbesondere auf einem externen Speichermedium sicherbar sind.

Nebst der Berechnung und Anzeige von Dosierungen und der Angabe der korrekten Größe von Atemwegs-Material und anderer Medizinalausrüstung, ist die erfindungsgemäße Einrichtung insbesondere auch dazu ausgebildet, ggf. auszuführende Techniken anhand von fixem oder bewegtem Instruktionsmaterial (z.B. in Form einer Abbildung oder eines Filmes) auf dem Anzeigemittel anzuzeigen. So kann insbesondere bei einer Beatmung und/oder einer Herzdruckmassage die Frequenz der Beatmung bzw. der Druckmassage angeben werden (z.B. auch akustisch verbal und/oder akustisch als Taktfrequenz), wobei die Angabe insbesondere altersentsprechend sowie entsprechend der Anzahl der Helfer erfolgen kann. Der bzw. die Helfer können somit genau instruiert bzw. im richtigen Rhythmus angeleitet werden.

Anstelle eines Maßbandes kann auch ein alternatives Messverfahren basierend auf einer Reflektions-Technik (z.B. Laser oder Ultraschall) verwendet werden.

Am Notfallkalkulator befindet sich dann eine abnehmbare Reflektionsplatte (siehe Figur 9). Diese wird am Kopf bündig angelegt. Vom sich am Fußende des Patienten befindlichen Notfallkalkulators wird der Laser bzw. Ultraschall zur am Kopf bündig angelegten Reflektionsplatte gesandt, dort reflektiert und zurück zum Notfallkalkulator gesandt. Darauf basierend wird die Körperlänge des Patienten ermittelt.

Die Berechnung von Gewicht und Alter des Patienten basierend auf der gemessenen Länge erfolgt mittels eines Algorithmus, wobei der Algorithmus geeignet in der Recheneinheit implementiert ist (z.B. in Form einer auf der Recheneinheit ausführbaren Software) wie folgt.

Für jede Ethnizität gibt es entsprechende Wachstumskurven (z.B. Schweizer, Deutsche oder Amerikanische) für Länge-Gewicht und für Länge-Alter. Diese Wachstumskurven sind geschlechtsspezifisch. Entsprechend der auf den Wachstumskurven dargestellten Perzentilen (3te, 10te, 25te, 50te, 75te, 90te und 97te) kann ein dem Habitus entsprechendes Gewicht einer Körperlänge zugeordnet werden. Bei der Errechnung des Alters wird bevorzugt die 50te Länge-Alter-Perzentile verwendet.

Bei der Gewichtseinschätzung wird die Länge-Gewichts-Perzentile bevorzugt in Abhängigkeit vom eingegebenen Habitus gewählt. So wird insbesondere die 50te Längen-Gewichtsperzentile bei normalem Habitus gewählt. Bei einem schlanken Habitus wird eine niedrigere Längen-Gewichtsperzentile gewählt (z.B. die 15te) und bei einem adipösen Habitus eine höhere Längen-Gewichts-Perzentile (z.B. die 85te). Verschiedene Länder (Deutschland, USA, Asien, etc.) haben verschieden Wachstumskurven für Länge-Gewicht und Länge-Alter. Sofern die Einrichtung die Angabe der Ethnizität des Patienten vorsieht, werden die Wachstumskurven, die der besagte Algorithmus verwendet, bevorzugt entsprechend der angegebenen Ethnizität ausgewählt.

Gemäß der Programmierung schlägt der besagte Algorithmus basierend auf den drei anzugebenden bzw. zu bestimmenden Parametern (Ethnizität, Geschlecht und Habitus) das Gewicht (insbesondere TKG, IKG und/oder LKG), das Alter (ohne Habituskorrektur), die KOF und den BMI für die gemessene Körperlänge vor.

Für die KOF (Körperoberfläche), den BMI, IKG und LKG verwendet der Algorithmus jeweils eine aus dem Stand der Technik bekannte und validierte Formel. Die beiden Formeln benötigen dabei als Eingabewerte das berechnete Gewicht und die gemessene Länge.

Alternativ zur Habitusauswahl kann es möglich sein, mittels des Maßbandes den Mitt-Oberarm-Umfang zu bestimmen. Basierend auf dieser Größe wird längen- und altersabhängig der BMI bzw. die zugehörige Mitt-Oberarm-Umfang-Perzentile berechnet. Dieser Wert wird dann zur Habituskorrektur der längenbasierten Gewichtsschätzung verwendet. So muss der Anwender keine Habitusschätzung mehr vornehmen.

### Beispiel einer Berechnung mittels Habitusauswahl:

Eine 102 cm große, weibliche Patientin mit Schweizer Herkunft mit schlankem Erscheinungsbild würde von CLAWAR auf ein Gewicht von 13.25 kg und einem Alter von 3.9 Jahre geschätzt werden. Das Programm würde in einer Reanimations-Situation folgende Medikamente und medizinische Materialen vorschlagen:
- Adrenalin 10 mcg/kg Körpergewicht (Konzentration: 100 mcg/ml): 130 mcg = 1.3 ml
- Atropin 20 mcg/kg Körpergewicht (Konzentration 500 mcg/ml): 250 mcg = 0.5 ml
- Beatmungsmaske: Größe 2
- Endotrachealer Tubus: 4 mm ID mit Cuff
- Oro-Pharyngealtubus: 60 mm
- Larynxmaske: Größe 2

### Beispiel einer Berechnung mittels Mitt-Oberarm-Umfang:

Eine 102 cm große, weibliche Patientin mit Schweizer Herkunft mit einem Mitt-Oberarmumfang von 18.8cm würde von CLAWAR auf ein Gewicht von 18.86 kg und einem Alter von 3.9 Jahren geschätzt werden. Das Programm würde in einer Reanimations-Situation folgende Medikamente und medizinische Materialen vorschlagen:
- Adrenalin 10 mcg/kg Körpergewicht (Konzentration: 100 mcg/ml): 190 mcg = 1.9 ml
- Atropin 20 mcg/kg Körpergewicht (Konzentration 500 mcg/ml): 500 mcg = 1 ml
- Beatmungsmaske: Größe 2
- Endotrachealer Tubus: 4 mm ID mit Cuff
- Oro-Pharyngealtubus: 60 mm
- Larynxmaske: Größe 2

Weiterhin kann die erfindungsgemäße Einrichtung zum Vitaldatenmonitoring verwendet werden.

Hierbei wird bevorzugt die Rechenleistung zur Messung eines EKG's, SPO₂, NIBP, IBP, Temperatur, ETCO₂, NIRS, BIS in entsprechenden Sensorkabeln und/oder in entsprechenden Sensoren integriert. Die Sensorkabel werden an der erfindungsgemäßen Einrichtung eingesteckt/konnektiert. Die erfindungsgemäße Einrichtung benötigt in einer besonders zu bevorzugenden Ausführung entsprechend bevorzugt nur noch ein entsprechendes Kabel mit gerätsentfernter Konnektion der Sensoren (d.h. insbesondere, dass das Kabel erst am Patienten in einzelne Stränge gesplittet wird und die Sensoren mit dem jeweils zugeordneten Strang verbunden werden, so dass nicht jeder Sensor mit einem eigenen Kabel an das Gerät herangeführt werden muss) bzw. die Konnektion findet via einer Wireless-Verbindung zwischen dem entsprechenden Sensor und der Einrichtung (z.B. Tablet-PC). Die erfindungsgemäße Einrichtung kann die Rechenleistung jedoch auch selber in herkömmlicher Weise bereitstellen. Da in einer Notfallstation (Ambulanz, Notfallabteilung) immer ein Vitaldatentransportmonitoring zum Einsatz kommt, ist die Integration des Vitaldaten-Monitoring in die erfindungsgemäße Einrichtung von Vorteil. Werden keine Daten aus der medizinischen Dosierungs- oder Materialberechnungs-Anwendung der Einrichtung benötigt, so kann das Anzeigemittel (Touchscreen) das Vitaldatenmonitoring in voller Größe anzeigen. Wird die Einrichtung zur Längenmessung sowie Gewichts-. Und Altersberechnung bzw. zur Dosierungs-/Materialberechnung gebraucht, so werden z.B. die Vitaldaten lediglich als Zahlenwerte im linken oder rechten oder oberen oder unteren Seitenbereich des Anzeigemittels angezeigt. Weiterhin können die von der Einrichtung errechneten Vitalgrenzwerte (z.B. niedrigster tolerierbarer Blutdruck, Herzfrequenz oder Sauerstoffsättigung) gleich vom Anzeigemittel und/oder einem externen Monitor übernommen werden sodass diese gleich die richtigen Alarmwerte automatisch gesetzt bekommen.

Werden die oben genannten Grenzwerte unterschritten ertönt vorzugsweise ein von der Einrichtung erzeugter akustischer und /oder visueller Alarm, der das medizinische Personal darauf hinweist, dass der Patient bedroht ist und gehandelt werden muss. Weiterhin kann die erfindungsgemäße Einrichtung grundsätzlich dazu eingerichtet sein, die auf dem Anzeigemittel angezeigte Information (insbesondere Vitaldatenmonitoring) bzw. das momentan angezeigte Bild über ein Kabel oder kabellos (Bluetooth, WIFI, etc.) auf ein zweites oder mehrere Displays (externer Monitor) zu spiegeln. Dies ist nützlich, um in einer Ambulanz oder in einem Schockraum die angezeigten Werten allen Beteiligten zugänglich zu machen.

Im Folgenden sollen spezielle Ausgestaltungen von Ausführungsformen anhand der Figuren beschrieben werden.

Diesbezüglich zeigen die Figuren 1 und 2 jeweils eine perspektivische Ansicht eines Ausführungsbeispiels einer erfindungsgemäßen Einrichtung 1, die ein Gehäuse 10 aufweist, in das eine Längenmesseinheit 20 integriert ist, die ein elektronisches Maßband 21 zum Messen der Länge des Patienten bzw. Kindes aufweist. Die ausgezogene Länge L wird mittels eines Anzeigemittels 50 angezeigt. Weiterhin kann mittels des Anzeigemittels 50 das durch die Einrichtung 1 berechnete Gewicht bzw. Alter des Patienten angezeigt werden. Die Einrichtung 1 bzw. das Anzeigemittel 50 kann zum Anzeigen der Länge des Gewichtes und des Alters jeweils eine separates Display aufweisen. Zum Berechnen des Gewichtes bzw. des Alters des Kindes bzw. Patienten (siehe auch oben) weist die Einrichtung 1 eine elektronische Recheneinheit 30 auf, die in das Gehäuse 10 integriert ist.

Weiterhin weist die Einrichtung 1 ein Eingabemittel 41 zum Eingeben des Geschlechts des Kindes, ein Eingabemittel 42 zum Eingeben des Habitus des Kindes sowie gegebenenfalls ein Eingabemittel 43 zum Eingeben der Ethnizität des Kindes (hier nicht gezeigt) auf. Wie oben bereits dargelegt, ist die elektronische Recheneinheit 30 dazu konfiguriert ist, einen oder mehrere dieser Parameter bei der Berechnung des Gewichtes zu verwenden.

Weiterhin kann die Einrichtung 1 ein Eingabemittel 45 aufweisen, mit dessen Hilfe die gemessene Länge, das berechnete Gewicht sowie das berechnete Alter korrigierbar ist. Die Einrichtung 1 kann dabei separate Eingabemittel zum korrigieren des Alters bzw. des Gewichts aufweisen.

Schließlich weist die Einrichtung 1 ein Eingabemittel 44 zum Quittieren der gemessenen Länge L auf. D.h., mittels des Maßbandes 21 der Längenmesseinheit 20 wird zunächst die Länge L (von Kopf bis Fuß) des Patienten gemessen und sodann die gemessene Länge L, die mittels des Anzeigemittels 50 anzeigbar ist, bestätigt. Auf diese Weise kann sichergestellt werden, dass eine korrekte Längenmessung zur Grundlage der folgenden Berechnungen (Alter und Gewicht etc.) gemacht wird. Das Eingabemittel 44 zum Quittieren der Längenmessung kann, wie in den Figuren 1 und 2 beispielhaft gezeigt, an dem Gehäuse 10 ausgebildet sein oder aber auch am freien Ende des ausziehbaren Maßbandes 21.

Die Figur 3 zeigt eine Abwandlung der in den Figuren 1 und 2 gezeigten Einrichtung 1. Hierbei ist wiederum die elektronische Recheneinheit 30 in das Gehäuse 10 der Einrichtung 1 integriert, ebenso wie die Längenmesseinheit 20, die hier zum Beispiel ein elektronisches Maßband 21 (siehe oben) aufweist. Weiterhin weist, wie im Zusammenhang mit den Figuren 1 und 2 beschrieben, das Gehäuse 10 die besagten Eingabemittel 41, 42, 44 auf. Im Unterschied zu den Figuren 1 und 2 ist nun aber vorgesehen, dass die Einrichtung 1 einen separaten Rechner 60 (hier nicht gezeigt) aufweist, bei dem es sich zum Beispiel um einen Tablet-PC oder ein Smartphone handeln kann, wobei dieser separate Rechner 60 (vgl. auch Figuren 14 bis 15) mithilfe eines geeigneten Mittels 12 am Gehäuse 10 der Einrichtung 1 lösbar festlegbar ist. Dieses Mittel 12 kann zum Beispiel eine geeignete Ausnehmung 12 aufweisen, in die der separate Rechner 60 einschiebbar ist. Die mittels der Längenmesseinheit 20 gemessene Länge L sowie die mittels der elektronischen Recheneinheit 30 berechneten Größen können dann über eine geeignete Verbindung, die in der Figur 3 zum Beispiel als Steckverbindung 24 ausgebildet ist, an den separaten Rechner 60 übertragen werden, der auf Basis des berechneten Alters und Gewichts (sowie ggf. KOF und BMI) alle Folgerechnungen (insbesondere Materialberechnungen) vornimmt..

Figur 4 zeigt eine weitere Ausführungsform einer erfindungsgemäßen Einrichtung 1, wobei hier die Einrichtung 1 ein Gehäuse 10 aufweist, das die oben beschriebenen Eingabemittel 41 42, 44 aufweist, die integrierte Längenmesseinheit 20 mit elektronischem Maßband 21 sowie ein in das Gehäuse 10 integriertes Anzeigemittel 50 in Form eines Displays, bei dem es sich insbesondere um einen Touchscreen handeln kann. Bei einer derartigen Ausführungsform kann es sich insbesondere um einen Tablet-PC handeln, in den zusätzlich die besagten Eingabemittel 41, 42, 44 sowie die Längenmesseinheit 20 integriert sind. Die elektronische Recheneinheit 30 kann hierbei durch den besagten Tablet-PC gebildet sein bzw. dessen Prozessor, so dass hier insbesondere keine gesonderte elektronische Recheneinheit vorzusehen ist.

Weiterhin zeigt Figur 5 eine Abwandlung der in der Figur 3 gezeigten Einrichtung 1. Hierbei ist wiederum die elektronische Recheneinheit 30 in das Gehäuse 10 der Einrichtung 1 integriert, ebenso wie die Längenmesseinheit 20, die hier zum Beispiel ein elektronisches Maßband 21 (siehe oben) aufweist. Weiterhin weist, wie im Zusammenhang mit Figuren 3 beschrieben, das Gehäuse 10 die besagten Eingabemittel 41, 42, 44 auf. Wie in Figur 3 ist es vorgesehen, dass die Einrichtung 1 einen separaten Rechner 60 (hier nicht gezeigt) aufweist, bei dem es sich zum Beispiel um einen Tablet-PC oder ein Smartphone handeln kann, wobei dieser separate Rechner 60 (vgl. auch Figuren 14 bis 15) mithilfe eines geeigneten Mittels 12 am Gehäuse 10 der Einrichtung 1 lösbar festlegbar ist. Dieses Mittel 12 kann zum Beispiel eine geeignete Ausnehmung 12 aufweisen, in die der separate Rechner 60 einschiebbar ist. Die mittels der Längenmesseinheit 20 gemessene Länge L sowie die mittels der elektronischen Recheneinheit 30 berechneten Größen können dann über eine geeignete kabellose Verbindung 25 (z.B. WiFi oder Bluetooth) an den separaten Rechner 60 übertragen werden, der auf Basis des berechneten Alters und Gewichts (sowie ggf. KOF und BMI) alle Folgerechnungen (insbesondere Materialberechnungen) vornimmt.

Weiterhin zeigt Figur 6 eine Ausführungsform einer erfindungsgemäßen Einrichtung 1, die einer Abwandlung der in der Figur 4 gezeigten Ausführungsform entspricht, wobei hier auf dem Gehäuse 10 nunmehr keine zusätzlichen Eingabemittel 41, 42, 44 vorgesehen sind, sondern diese durch das Anzeigemittel 50 gebildet werden, das hier als Touchscreen ausgebildet ist. Die einzelnen Eingabemittel können hierbei zum Beispiel als Icons auf dem Touchscreen dargestellt werden.

Die Figur 7 zeigt eine weitere Ausführungsform einer erfindungsgemäßen Einrichtung 1, die ebenfalls einer Abwandlung der in der Figur 4 gezeigten Ausführungsform entspricht. Im Unterschied zur Figur 4 ist hier keine in das Gehäuse 10 integrierte Längenmesseinheit 20 vorgesehen, sondern eine separate Längenmesseinheit 20, die zum Beispiel durch ein analoges Maßband 20 gebildet sein kann. Am Gehäuse 10 ist nun zusätzlich gegenüber der Ausführungsform gemäß Figur 4 ein Eingabemittel 46 vorgesehen, mit dessen Hilfe die mittels des Maßbandes 20 (oder einer sonstigen Längenmesseinheit) gemessene Länge des Patienten manuell eingegeben werden kann.

Die Figur 8 zeigte des Weiteren eine Abwandlung der in der Figur 7 gezeigten Einrichtung 1, wobei hier sämtliche Eingabemittel 41, 42, 46 durch das Anzeigemittel 50, das hier als ein Touchscreen ausgebildet ist, gebildet sind. Die Einrichtung 1 kann also insbesondere in Form eines Tablet-PCs oder in Form eines Smartphones vorliegen, in den bzw. in das die mittels einer separaten Längenmesseinheit 20 gemessene Länge des Kindes bzw. Patienten eingegeben wird.

Weiterhin zeigt die Figur 9 eine Abwandlung der in den Figuren 1 und 2 gezeigten erfindungsgemäßen Einrichtung 1, wobei hier anstelle eines elektronischen Maßbandes die Längenmesseinheit 20 eine Laserquelle oder eine Ultraschallquelle verwendet, die in das Gehäuse 10 integriert ist, um die Länge L des Kindes bzw. des Patienten zu messen. Hierbei ist insbesondere ein Reflektor 23, zum Beispiel in Form einer Reflexionsplatte, vorgesehen, der an ein Körperende des Patienten gehalten werden kann, sodass dort das Laserlicht bzw. der Ultraschall zurück zum Gehäuse 10 reflektierbar ist. Vorzugsweise ist vorgesehen, dass der besagte Reflektor 23 am Gehäuse 10 der Einrichtung 1 lösbar festgelegt war ist, sodass er auf einfache Art und Weise mit dem Gehäuse 10 transportierbar ist.

Weiterhin zeigt die Figur 10 eine erfindungsgemäße Einrichtung 1, bei der es sich zum Beispiel um eine der Einrichtungen gemäß Figuren 3 bis 8 handeln kann, wobei hier die Einrichtung 1 auch dazu ausgebildet ist, Vitaldaten des Patienten, dessen Länge zu messen ist, zu überwachen (siehe auch oben). Hierbei kann gemäß Figur 10 - solange keine Berechnungen basierend auf der Länge des Patienten dargestellt werden - der gesamte Bildschirm des Anzeigemittels 50 dazu verwendet werden, die Vitaldaten des Patienten anzuzeigen. Für den Fall, dass das Anzeigemittel 50 auch dazu benötigt wird, auf der Länge des Patienten basierende Berechnungen anzuzeigen bzw. berechnete Medikamentendosierungen oder Materialmengen anzuzeigen, besteht die Möglichkeit, die Anzeige der Vitaldaten des Patienten zu verkleinern, wie es in der Figur 11 angedeutet ist, sodass entsprechend das Anzeigemittel 50 auch zur Anzeige von anderen Informationen verwendbar ist. Grundsätzlich können in allen Ausführungsformen der Erfindung Vitaldaten des Patienten überwacht werden, sofern die Einrichtung 1 über ein entsprechendes bzw.-geeignetes Anzeigemittel 50 verfügt.

Weiterhin zeigt die Figur 12 eine Draufsicht auf eine erfindungsgemäße Einrichtung 1 nach Art der Figur 4, wobei hier im Unterschied zur Figur 4 die Längenmesseinheit 20 nicht zwingend ein elektronisches Maßband aufweist, sondern ein mechanisches bzw. analoges Maßband 21, das aus dem Gehäuse 10 der Einrichtung 1 ausziehbar ist. Hierbei ist zum Ablesen der ausgezogenen Länge L des Maßbandes 21 am Gehäuse 10 der Einrichtung ein Sichtfenster 11 vorgesehen, an dem das Maßband 21 beim Ausziehen vorbei läuft, so dass die ausgezogene Länge durch das Sichtfenster 11 hindurch auf dem Maßband 21 selbst ablesbar ist. Es kann sich aber auch um ein elektronisches Maßband handeln, bei welchem im Falle eines Funktionsausfalles die Länge durch das Sichtfenster 11 abgelesen werden kann. Zum genaueren Ablesen der ausgezogenen Länge L kann am Gehäuse 10 benachbart zum Sichtfenster 11 eine entsprechende Markierung vorgesehen sein. Weiterhin weist das Gehäuse 10 nunmehr ein Eingabemittel 46 auf, mit dessen Hilfe die analog gemessene Länge L des Kindes bzw. Patienten in die Einrichtung 1 eingebbar ist.

Figur 13 zeigt eine Abwandlung der in der Figur 12 gezeigten Ausführungsform, wobei hier sämtliche Eingabemittel 41, 42, 44, 46 durch das Anzeigemittel 50 der Einrichtung 1 gebildet sind, das hier als ein Touchscreen ausgebildet ist.

Weiterhin zeigt Figur 14 eine Abwandlung der in der Figur 12 gezeigten Ausführungsform, wobei hier (wie bereits oben beschrieben) die besagten Eingabemittel 41, 42, 44, 46 separat am Gehäuse 10 ausgebildet sind und das Gehäuse 10 ferner dazu konfiguriert ist, einen separaten Rechner 60 aufzunehmen, der das Anzeigemittel 50 aufweist (z.B. Touchscreen), wobei es sich bei dem Rechner 60 bevorzugt um ein Smartphone oder einen Tablet-PC handelt. Hierzu kann das Gehäuse eine Ausnehmung 12 zum lösbaren Festlegen bzw. Aufnehmen des Rechners 60 aufweisen (siehe auch oben). Wie oben bereits erläutert, kann eine Datenübertragungsverbindung zwischen dem Rechner 30 und der elektronischen Recheneinheit 30 mittels einer Steckverbindung 24 (oder mittels einer kabellosen Verbindung) realisiert werden.

Schließlich zeigt Figur 15 eine Abwandlung der in der Figur 14 gezeigten Ausführungsform, wobei hier wiederum sämtliche Eingabemittel 41, 42, 44, 46 durch das Anzeigemittel 50 des separaten Rechners 60 gebildet sind, wobei es sich bei dem Anzeigemittel 50 um einen Touchscreen handelt.

Abschließend sollen anhand der Figuren 16A bis 16C drei grundsätzliche Anordnungs- bzw. Konfigurationsmöglichkeiten in einer Übersicht dargestellt werden, wobei die einzelnen Konfigurationen bereits oben im Detail beschrieben worden sind.

Gemäß Figur 16A kann eine erfindungsgemäße Einrichtung eine Längenmesseinheit 20 mit einem in das Gehäuse PEC (kurz für pediatric emergency calulator) bzw. 10 integrierten (digitales) Maßband oder eine entsprechend integrierte laser- oder ultraschallbasierte Längenmesseinheit 20 aufweisen. Hierbei ist auch eine manuelle Eingabe der gemessenen Länge optional über ein Ablesefenster/Sichtfenster 11 möglich.

Das Anzeigemittel 50 kann hierbei fest mit dem Gehäuse PEC der Einrichtung verbunden sein und eine mechanische Einheit bilden, kann jedoch auch mittels Einschub (z.B. Rahmen) oder als isolierte Einheit via Schnittstelle (WiFi, Bluetooth, Kabel) verbunden werden.

Figur 16B zeigt diese möglichen Ausformungen in Zusammenhang mit einer Längenmesseinheit 20, die ein integriertes mechanisches Maßband aufweist. Die Länge wird hier über ein Ablesefenster/Sichtfenster 11 ermittelt und manuell eingegeben.

Figur 16C zeigt die möglichen Ausformungen im Zusammenhang mit einer Längenmesseinheit 20, bei der es sich im Unterscheid zu den Figuren 16A und 16B um ein externes mechanisches Maßband handelt. Hier wird die Länge konventionell ermittelt und manuell eingegeben.

Bei all diesen Konfigurationen kann gemäß Figur 17 die Eingabe der notwendigen Daten über Eingabemittel auf dem Gehäuse 10 (außerhalb des Anzeigemittels/Displays 50) erfolgen, hier als "NUR auf dem PEC" gekennzeichnet, bis hin zu einer Konfiguration, bei der alle Eingabemittel durch das Anzeigemittel/Display 50 gebildet werden (Touchscreen), hier gekennzeichnet als "NUR auf dem Display" möglich sein. Dazwischen sind alle möglichen Mischformen ebenfalls möglich. Je nach Bedarf können einzelne der hierin beschriebenen Eingabemittel separat zum Anzeigemittel 50 vorgesehen werden oder können durch dieses bereitgestellt werden (z.B. als Touchscreenfunktion)

## Patentansprüche

1. Einrichtung (1) zur Berechnung des Gewichtes und des Alters eines Kindes, insbesondere in einer medizinischen Notfallsituation, wobei die Einrichtung (1) aufweist:
- ein Gehäuse (10);
- eine Längenmesseinheit (20), die zum Messen der Länge des Körpers des Kindes ausgebildet ist;
- eine im Gehäuse (10) angeordnete elektronische Recheneinheit (30), die dazu konfiguriert ist, unter Verwendung der gemessenen Länge eine automatische Berechnung des Gewichtes und/oder des Alters des Kindes vorzunehmen.

2. Einrichtung nach Anspruch 1, **dadurch gekennzeichnet, dass** die Längenmesseinheit (20) in das Gehäuse (10) integriert ist.

3. Einrichtung nach Anspruch 1 oder 2, **dadurch gekennzeichnet, dass** die Einrichtung Eingabemittel (41, 42) zum Eingeben des Geschlechts und des Habitus aufweist, sowie vorzugsweise auch ein Eingabemittel zum Eingeben der Ethnizität des Kindes, wobei die elektronische Recheneinheit (30) dazu konfiguriert ist, einen oder mehrere der folgenden Parameter bei der Berechnung des Gewichtes zu verwenden: das eingegebene Geschlecht, den eingegebenen Habitus, die eingegebene Ethnizität.

4. Einrichtung nach einem der vorhergehenden Ansprüche, **dadurch gekennzeichnet, dass** die Einrichtung (1) ein Eingabemittel (44) aufweist, das zum Quittieren der gemessenen Länge durch den Anwender ausgebildet ist.

5. Einrichtung nach einem der vorhergehenden Ansprüche, **dadurch gekennzeichnet, dass** die Einrichtung ein Eingabemittel (45) zum Korrigieren der gemessenen Länge und/oder des berechneten Gewichtes und/oder des berechneten Alters aufweist.

6. Einrichtung nach einem der vorhergehenden Ansprüche, **dadurch gekennzeichnet,** das die Einrichtung (1) ein Eingabemittel (46) zum Eingeben der mittels der Längenmesseinheit (20) gemessenen Länge (L) aufweist.

7. Einrichtung nach einem der vorhergehenden Ansprüche, **dadurch gekennzeichnet, dass** die Einrichtung (1) ein Anzeigemittel (50) zum Anzeigen der gemessenen Länge sowie des berechneten Gewichtes und/oder des berechneten Alters aufweist, wobei das Anzeigemittel (50) insbesondere als Touchscreen ausgebildet ist.

8. Einrichtung nach Anspruch 7 sowie nach zumindest einem der Ansprüche 3 bis 6, **dadurch gekennzeichnet, dass** das Anzeigemittel (50) sowie die Eingabemittel (41, 42, 43, 44, 45) am Gehäuse (10) vorgesehen sind.

9. Einrichtung nach einem der Ansprüche 1 bis 7, **dadurch gekennzeichnet, dass** die Einrichtung (1) einen separaten Rechner (60) aufweist, der das Anzeigemittel (50) aufweist, wobei die elektronische Recheneinheit (30) dazu ausgebildet ist, eine oder mehrere der folgenden Informationen auf den separaten Rechner (60) zu übertragen: die gemessene Länge, das berechnete Gewicht, das berechnete Alter, das eingegebene Geschlecht des Kindes, den eingegebenen Habitus des Kindes, die eingegebene Ethnizität des Kindes.

10. Einrichtung nach Anspruch 9, **dadurch gekennzeichnet, dass** die Einrichtung (1) dazu konfiguriert ist, die besagten Informationen über eine Kabelverbindung, Steckverbindung oder eine drahtlose Verbindung auf den separaten Rechner (60) zu übertragen.

11. Einrichtung nach Anspruch 9 oder 10, **dadurch gekennzeichnet, dass** das Gehäuse (10) ein Mittel (12) zum Festlegen des separaten Rechners (60) am Gehäuse (10) aufweist, wobei das Mittel (12) insbesondere eine Ausnehmung oder einen Rahmen zur Aufnahme des separaten Rechners (60) aufweist.

12. Einrichtung nach zumindest einem der Ansprüche 3 bis 6 und nach Anspruch 7 oder einem der Ansprüche 8 bis 11 sofern rückbezogen auf Anspruch 7, **dadurch gekennzeichnet, dass** jedes der folgenden Eingabemittel ein separat zum Anzeigemittel (50) ausgebildete Eingabemittel ist, das am Gehäuse (10) vorgesehen ist, oder ein durch das Anzeigemittel (50) gebildetes Eingabemittel:
- das Eingabemittel (41) zum Eingeben des Geschlechts,
- das Eingabemittel (42) zum Eingeben des Habitus,
- das Eingabemittel zum Eingeben der Ethnizität,
- das Eingabemittel (44) zum Quittieren der Messung,
- das Eingabemittel (45) zum Korrigieren der Länge und/oder des Gewichtes und/oder des Alters,
- das Eingabemittel (46) zum Eingeben der gemessenen Länge.

13. Einrichtung nach einem der vorhergehenden Ansprüche, **dadurch gekennzeichnet, dass** die Einrichtung (1), insbesondere die Recheneinheit (30) oder der separate Rechner (60), dazu konfiguriert ist, eine Dosierungsberechnung eines dem Kind zu verabreichenden Medikaments und/oder eine Materialberechnung durchzuführen und die berechnete Dosis sowie das berechnete Material anzuzeigen, wobei insbesondere die Einrichtung (1), insbesondere die Recheneinheit (30) oder der separate Rechner (60), ferner dazu konfiguriert ist, für die Dosierungsberechnung oder die Materialberechnung eine, mehrere oder sämtliche der folgenden Informationen zu verwenden: die gemessene Länge, das berechneten Gewicht, das berechnete Alter, das Geschlecht des Kindes, den Habitus des Kindes, die Ethnizität des Kindes.

14. Einrichtung nach einem der vorhergehenden Ansprüche, **dadurch gekennzeichnet, dass** die Längenmesseinheit (20) ein ausziehbares Maßband aufweist (21), das aus dem Gehäuse (10) ausziehbar ist, wobei insbesondere
- die Längenmesseinheit (20) dazu konfiguriert ist, die aus dem Gehäuse (10) ausgezogene Länge (L) des Maßbandes (21) automatisch zu bestimmen, und/oder
- wobei am Gehäuse (10) ein Sichtfenster (11) vorgesehen ist, an dem die ausgezogene Länge (L) ablesbar ist.

15. Einrichtung nach einem der Ansprüche 1 bis 13, **dadurch gekennzeichnet, dass** die Längenmesseinheit (20) zum Messen der Länge des Kindes einen Laser (22) oder eine Ultraschallquelle (22) aufweist, sowie insbesondere einen lösbar am Gehäuse festlegbaren Reflektor (23) zum Reflektieren des Laserlichts oder des Ultraschalls.

16. Einrichtung nach einem der vorangehenden Ansprüche, **dadurch gekennzeichnet, dass** die Einrichtung (1) dazu ausgebildet ist, Patientenvitaldaten anzuzeigen, wobei insbesondere die Einrichtung ein Verbindungsmittel zum Herstellen einer Datenübertragungsverbindung zwischen zumindest einem Sensor zum Messen von Patientenvitaldaten und der Einrichtung (1) aufweist.
